Europäisches Patentamt

European Patent Office

Office européen des brevets

(19)

(11) Veröffentlichungsnummer : **0 442 075 B1**

## (12) EUROPÄISCHE PATENTSCHRIFT

(45) Veröffentlichungstag der Patentschrift :
11.08.93 Patentblatt 93/32

(51) Int. Cl.⁵ : **C07C 19/08, C07C 17/00**

(21) Anmeldenummer : 90123784.2

(22) Anmeldetag : 11.12.90

(54) **Verfahren zur Herstellung von 1,1,1,3,3,3-Hexafluorpropan und 2-Chlor-1,1,1,3,3,3-hexafluorpropan.**

(30) Priorität : 14.02.90 DE 4004495

(43) Veröffentlichungstag der Anmeldung :
21.08.91 Patentblatt 91/34

(45) Bekanntmachung des Hinweises auf die
Patenterteilung :
11.08.93 Patentblatt 93/32

(84) Benannte Vertragsstaaten :
BE DE ES FR GB IT NL

(56) Entgegenhaltungen :
EP-A- 0 349 115
DE-B- 1 154 797
DE-B- 1 246 703
THE JOURNAL OF ORGANIC CHEMISTRY,
Band 28, 1963, Easton J.T. MAYNARD "The
synthesis of highly fluorinated compounds by
use of potassium fluoride in polar solvents",
Seiten 112-115

(56) Entgegenhaltungen :
CHEMICAL ABSTRACTS, Band 81, Nr. 1, 8. Juli
1974, Columbus, Ohio, USA; N. P. AKTAEV et
al. "Bis(trifluoromethyl)methane", Seite 267,
Zusammenfassung Nr.3 329x
THE JOURNAL OF ORGANIC CHEMISTRY,
Band 54, 1989, Easten M. HANACK et al.
"Facile synthesis of trifluoro- and hexafluoroisopropyl halides", Seite 1432-1435

(73) Patentinhaber : BAYER AG
W-5090 Leverkusen 1 Bayerwerk (DE)

(72) Erfinder : Gassen, Karl-Rudolf, Dr.
Auenweg 6a
W-5068 Odenthal (DE)
Erfinder : Bielefeldt, Dietmar, Dr.
Beuthener Strasse 13
W-4030 Ratingen 6 (DE)
Erfinder : Marhold, Albrecht, Dr.
Carl-Duisberg-Strasse 329
W-5090 Leverkusen 1 (DE)
Erfinder : Schwarz, Hans-Helmut, Dr.
Rather Strasse 90
W-4150 Krefeld (DE)

## Beschreibung

Bisher sind die im Titel genannten Hexafluorpropane nur nach aufwendigen Verfahren, unter Einsatz von schlecht zugänglichen Ausgangsmaterialien und/oder in schlechten Ausbeuten erhältlich. So ist in J. Org. Chem. 28, 112 (1963) beschrieben, daß man aus Hexachlorpropan und Pentachlorpropen durch Umsetzung mit Kaliumfluorid in Gegenwart eines polaren Lösungsmittels 1,1,1,3,3,3-Hexafluorpropan nur in etwa 20 %iger Ausbeute erhalten kann.

J. Org. Chem. 54, 1432 (1989) beschreibt die Herstellung von 2-Chlor-1,1,1,3,3,3-hexafluorpropan aus 1,1,1,3,3,3-Hexafluor-2-propanol, das zunächst in die entsprechende Nonaflat-Verbindung (d.i. ein Nonafluorbutansulfonat) überführt wird, in die man dann mit Lithiumchlorid in Gegenwart eines Kronenethers ein Chloratom in die 2-Stellung einführt.

1,1,1,3,3,3-Hexafluorpropan und 2-Chlor-1,1,1,3,3,3-hexafluorpropan gewinnen zunehmend an technischem Interesse - die erstere Verbindung als nicht die Ozonschicht der Atmosphäre gefährdendes Treibmittel (siehe Bild der Wissenschaften 2, 49 (1988)) und die letztere Verbindung als Wärmetauscher-Flüssigkeit (siehe EP-A 72 308). Es besteht deshalb das Bedürfnis nach einem technisch vorteilhaften Herstellungsverfahren für diese Stoffe.

Es wurde nun ein Verfahren zur Herstellung von Hexafluorpropanen der Formel (I) gefunden

$$CF_3\text{-}CHX\text{-}CF_3 \qquad (I)$$

in der

X   für Wasserstoff oder Chlor steht,

das dadurch gekennzeichnet ist, daß man Hexachlorpropen mit Fluorwasserstoff in der Gasphase in Gegenwart eines Katalysators umsetzt.

Das Ausgangsmaterial Hexachlorpropen steht kostengünstig zur Verfügung, da es aus einfachen Grundchemikalien (z.B. aus Chloroform und Tetrachlorethan) erhältlich ist.

Als Fluorwasserstoff kommt handelsüblicher Fluorwasserstoff in Frage. Er kann als solcher eingesetzt werden, aber auch in verdünnter Form, beispielsweise gemischt mit Stickstoff.

Erfindungsgemäß werden beide Reaktanden in dar Gasphase umgesetzt. Geeignete Reaktionstemperaturen sind beispielsweise solche im Bereich von 250 bis 600°C. Vorzugsweise arbeitet man im Bereich von 300 bis 550°C. insbesondere im Bereich 350 bis 500°C.

Das erfindungsgemäße Verfahren kann bei beliebigen Drucken in der Reaktionszone durchgeführt werden, soweit die Reaktanden beim jeweils gewählten Druck in der Gasphase verbleiben. Vorzugsweise arbeitet man bei 1 bis 3 bar, insbesondere bei Normaldruck oder einem Überdruck, der dem Strömungswiderstand der verwendeten Apparatur entspricht.

Die relativen Mengen an eingesetztem Hexachlorpropen und Fluorwasserstoff können in weiten Grenzen variiert werden. Es ist von Vorteil, den Fluorwasserstoff im Überschuß einzusetzen, beispielsweise auf 1 Mol Hexachlorpropen 5 bis 100 Mol Fluorwasserstoff. Besonders bevorzugt setzt man auf 1 Mol Hexachlorpropen 10 bis 50 Mol Fluorwasserstoff ein.

Als Katalysatoren für das erfindungsgemäße Verfahren kommen z.B. Halogenide und Oxide von Metallen und Übergangsmetallen in Frage. Geeignet sind insbesondere Chloride, Fluoride und/oder gegebenenfalls gemischte Oxide von Kupfer, Chrom, Eisen, Wismut, Zink, Lanthan, Cer, Zirkon, Vanadium, Molbydän, Wolfram und/oder Platin. Bevorzugt sind Chrom(III)-salze alleine oder im Gemisch mit den genannten Metallchloriden, -fluoriden und/oder -oxiden. Die Katalysatoren können als solche, z.B. in stückiger Form, verwendet werden, aber auch aufgebracht auf einen Träger, beispielsweise auf Aluminiumoxid, Magnesiumoxid, Magnesiumfluorid, Calciumfluorid, Zinkchlorid und/oder Aktivkohle.

Besonders bevorzugt sind Chrom(III)-salze, insbesondere Chrom(III)-fluoride, Chrom(III)-chloride und Chrom(III)-oxide auf einem der genannten Trägermaterialien.

Die Strömungsgeschwindigkeit des Reaktionsgemisches und die Katalysatormenge kann man beispielsweise so wählen, daß sich Katalysatorbelastungen von 50 bis 1.000 g/l·h, vorzugsweise 150 bis 500 g/l·h ergeben.

Als Werkstoffe für die Reaktions- und Nebenapparate kommen gegen den Angriff von Fluor- und Chlorwasserstoff auch bei hohen Temperaturen widerstandsfähige Materialien in Frage, beispielsweise Nickel-, Chrom- und/oder Molybdänstähle, sowie reines Nickel.

Man kann die erfindungsgemäße Reaktion z.B. so durchführen, daß man die Ausgangsmaterialien vereinigt oder getrennt auf Reaktionstemperatur erhitzt, dann einer Reaktionszone zuführt (z.B. einem beheizbaren Rohr, das den Katalysator enthält), das die Reaktionszone verlassende Gasgemisch gegebenenfalls wäscht und abkühlt, so daß zumindest die organischen Bestandteile kondensieren und diese gegebenenfalls durch Destillation weiter auftrennt und reinigt.

Nach der Durchführung des erfindungsgemäßen Verfahrens erhält man im allgemeinen Gemische aus Fluor- und/oder Chlor enthaltenden Propanen und Propenen. Die Propene kann man in die erfindungsgemäße Umsetzung recyclisieren.

Die Zusammensetzung des die Reaktionszone verlassenden Gemisches kann man durch die Reaktionstemperatur beeinflussen. Man erhält besonders hohe Anteile an 1,1,1,3,3,3-Hexafluorpropan (Formel (I), X = Wasserstoff) wenn man die Reaktion bei relativ hohen Temperaturen durchführt, beispielsweise bei 435 bis 525°C, insbesondere bei 450 bis 500°C. Man erhält besonders hohe Anteile an 2-Chlor-1,1,1,3,3,3-hexafluorpropan, wenn man die Reaktion bei relativ tiefen Temperaturen durchführt, beispielsweise bei 325 bis 415°C, insbesondere bei 350 bis 400°C.

Das erfindungsgemäße Verfahren gestattet die Herstellung von Hexafluorpropanen der Formel (I) auf einfache und preisgünstige Weise. Bei Rückführung der im Reaktionsgemisch vorhandenen halogenierten Propene lassen sich im allgemeinen Hexafluorpropane der Formel (I) in Ausbeuten von über 80 %, bezogen auf eingesetztes Hexachlorpropen erhalten. Aus dem Reaktionsgemisch kann man gegebenenfalls $CF_3$-$CCl_2$-$CF_3$ und/oder $CF_3$-$CHCl$-$CF_3$ abtrennen und gegebenenfalls separat und gegebenenfalls im Gemisch mit 1,1,1,3,3,3-Hexafluorpropan katalytisch zu 1,1,1,3,3,3-Hexafluorpropan hydrieren.

Diese katalytische Hydrierung kann man auf an sich bekannte Weise durchführen, beispielsweise indem man ein Gemisch aus Wasserstoff und $CF_3$-$CCl_2$-$CF_3$ und/oder $CF_3$-$CHCl$-$CF_3$ über einen fest angeordneten Hydrierkatalysator leitet. Das Molverhältnis hydrierbarer Verbindungen zu Wasserstoff kann dabei beispielsweise 1:3 bis 1:50 betragen. Vorzugsweise beträgt es 1:4 bis 1:20.

Die Hydrierung kann beispielsweise bei Normaldruck oder bei erhöhten Drucken, beispielsweise im Bereich von Normaldruck bis 20 bar durchgeführt werden. Vorzugsweise wird sie bei Normaldruck durchgeführt.

Als Hydrierkatalysatoren kommen insbesondere solche in Frage, die Übergangsmetalle auf Trägermaterialien enthalten. Von den Übergangsmetallen sind Palladium und Platin bevorzugt, insbesondere Palladium. Beispiele für Trägermaterialien sind Aktivkohlen, Aluminiumoxide, Siliciumdioxide, Bariumsulfat, Spinelle, Silikate und Titandioxid. Bevorzugt sind Aktivkohlen und Lithium-Aluminium-Spinelle. Die Katalysatoren können beispielsweise 0,5 bis 30 g Übergangsmetall pro Liter Trägermaterial enthalten. Vorzugsweise liegt dieser Gehalt im Bereich 2 bis 20 g/l.

Die Strömungsgeschwindigkeit des Hydriergemisches und die Katalysatormenge kann beispielsweise so gewählt werden, daß sich Katalysatorbelastungen von 10 bis 1000 g/l·h ergeben, vorzugsweise solche von 50 bis 500 g/l·h. Die Reaktionstemperaturen liegen im allgemeinen über 20°C, bevorzugt im Bereich 100 bis 250°C.

Das bei der Hydrierung entstehende Gemisch kann beispielsweise aufgearbeitet werden, indem man es zur Entfernung des entstandenen Chlorwasserstoffs mit Wasser oder verdünnter Lauge wäscht und die gasförmigen Produkte, gegebenenfalls nach Trocknung, kondensiert.

## Beispiele

### Beispiele 1 bis 4

Durch ein Nickelrohr, das 750 ml eines gemäß Beispiel 8 hergestellten Katalysators enthielt wurden bei der jeweils angegebenen Temperatur pro Stunde 40 g Hexachlorpropen, 80 g Fluorwasserstoff und 1 l Stickstoff geleitet. Das die Reaktionszone verlassende Gasgemisch wurde mit Wasser gewaschen, getrocknet und die kondensierbaren Anteile bei -78°C kondensiert.

Der Gehalt an organischen Bestandteilen im Kondensat wurde gaschromatographisch und mittels Kernresonanzspektroskopie bestimmt. Es ergaben sich folgende Ergebnisse:

| Zusammensetzung des isolierten organischen Produkts (Gew.-%) | Sdp. (°C) | Beispiel Nr. und Reaktionstemperatur | | | |
|---|---|---|---|---|---|
| | | 1 350°C | 2 400°C | 3 450°C | 4 500°C |
| $CF_3-CH_2-CF_3$ | -0,7 | - | 10 | 55 | 64 |
| $CF_3-CHCl-CF_3$ | 16 | 60 | 69 | 23 | 15 |
| $CF_3-CCl_2-CF_3$ | 33-34 | 1 | 3 | 6 | 7 |
| $CF_3-CCl=CF_2$ | 5 | 9 | 9 | 6 | 6 |
| $CF_3-CCl=CFCl$ | - | 15 | 5 | 6 | 4 |
| $CF_3-CCl=CCl_2$ | 89-91 | 15 | 4 | 4 | 4 |

Die einzelnen Bestandteile lassen sich gewünschtenfalls leicht durch Destillation auftrennen.

Beispiel 5 bis 7

Ein senkrecht angeordneter, elektrisch beheizbarer Rohrreaktor aus Quarz (Länge 310 mm, Durchmesser 36 mm) wurden mit 200 ml eines Trägerkatalysators beschickt, der 18 g Palladium pro Liter eines kugelförmigen Lithium-Aluminium-Spinells (Kugeldurchmesser 3 bis 5 mm) enthielt.

Der Katalysator wurde 6 Stunden lang bei 250°C unter Durchleiten von 20 bis 25 ml Wasserstoff pro Stunde konditioniert. Danach wurden jeweils die unten beschriebenen Hydrierungen durchgeführt. Die das Quarzrohr

4

verlassenden Gase wurden bei -78°C kondensiert und mit $^{19}$F-NMR-Spektroskopie untersucht.

Beispiel 5

Einsätze: 0,16 Mol/h $CF_3$-CHCl-$CF_3$ und 2,5 Mol/h Wasserstoff
Reaktionsbedingungen: 200°C, Normaldruck
Katalysatorbelastung: 150 g/l·h
$CF_3$-$CH_2$-$CF_3$ wurde mit einem Umsatz von 95 % und in einer Selektivität von 94 % erhalten.

Beispiel 6

Einsätze: 0,2 Mol/h $CF_3$-CHCl-$CF_3$ und 0,8 Mol/h Wasserstoff
Reaktionsbedingungen: 200°C, Normaldruck
Katalysatorbelastung: 180 g/l·h
$CF_3$-$CH_2$-$CF_3$ wurde mit einem Umsatz von 92 % und in einer Selektivität von 85 % erhalten.

Beispiel 7

Einsätze: 0,21 Mol/h $CF_3$-$CCl_2$-$CF_3$ und 1,1 Mol/h Wasserstoff
Reaktionsbedingungen: 200°C, Normaldruck
Katalysatorbelastung: 200 g/l·h
$CF_3$-$CH_2$-$CF_3$ wurde mit einem Umsatz von 89 % und in einer Selektivität von 87 % erhalten.

Beispiel 8

300 g $CrCl_3$ · 6 $H_2O$ und 30 g $MgF_2$ wurden in 10 l Wasser auf 90°C erwärmt. Nach 1 Stunde wurden 1300 g einer 11 %igen wäßrigen Ammoniaklösung zudosiert. Anschließend wurde 1 Stunde nachgerührt, abkühlen gelassen und der ausgefallene Feststoff über eine Filternutsche abfiltriert. Der Feststoff wurde 2 mal mit Wasser gewaschen, getrocknet, pulverisiert und mit 2 Gew.-% Graphit homogen vermischt. Dieses Gemisch wurde zu 4 mm großen Tabletten gepreßt.

**Patentansprüche**

1. Verfahren zur Herstellung von Hexafluorpropanen der Formel (I)
$$CF_3\text{-CHX-}CF_3 \qquad (I),$$
in der
X für Wasserstoff oder Chlor steht,
dadurch gekennzeichnet, daß man Hexachlorpropen mit Fluorwasserstoff in der Gasphase in Gegenwart eines Katalysators umsetzt.

2. Verfahren nach Anspruch 1, dadurch gekennzeichnet, daß man die Umsetzung bei 250 bis 600°C durchführt.

3. Verfahren nach Ansprüchen 1 und 2, dadurch gekennzeichnet, daß man auf 1 Mol Hexachlorpropen 5 bis 100 Mol Fluorwasserstoff einsetzt.

4. Verfahren nach Ansprüchen 1 bis 3, dadurch gekennzeichnet, daß man als Katalysatoren Halogenide und/oder Oxide von Metallen und/oder Übergangsmetallen einsetzt.

5. Verfahren nach Ansprüchen 1 bis 4, dadurch gekennzeichnet, daß man Chrom(III)-salze enthaltende Katalysatoren einsetzt.

6. Verfahren nach Ansprüchen 1 bis 5, dadurch gekennzeichnet, daß man zur Herstellung von 1,1,1,3,3,3-Hexafluorpropen die Reaktion bei 435 bis 525°C durchführt.

7. Verfahren nach Ansprüchen 1 bis 6, dadurch gekennzeichnet, daß man zur Herstellung von 2-Chlor-1,1,1,3,3,3-hexafluorpropan das Verfahren bei 325 bis 415°C durchführt.

8. Verfahren nach Ansprüchen 1 bis 7, dadurch gekennzeichnet, daß man aus dem Reaktionsgemisch abgetrenntes CF$_3$-CCl$_2$-CF$_3$ und/oder CF$_3$-CHCl-CF$_3$ katalytisch zu 1,1,1,3,3,3-Hexafluorpropan hydriert.

9. Verfahren nach Anspruch 8, dadurch gekennzeichnet, daß man als Hydrierkatalysatoren Übergangsmetalle auf Trägermaterialien verwendet.

**Claims**

1. Process for the preparation of hexafluoropropanes of the formula (1)
$$CF_3\text{-}CHX\text{-}CF_3$$
in which
X represents hydrogen or chlorine,
characterised in that hexachloropropene is reacted with hydrogen fluoride in the gas phase in the presence of a catalyst.

2. Process according to Claim 1, characterised in that the reaction is carried out at 250 to 600°C.

3. Process according to Claims 1 and 2, characterised in that 5 to 100 mol of hydrogen fluoride are used per 1 mol of hexachloropropene.

4. Process according to Claims 1 to 3, characterised in that halides and/or oxides of metals and/or transition metals are used as catalysts.

5. Process according to Claims 1 to 4, characterised in that catalysts containing chromium(III) salts are used.

6. Process according to Claims 1 to 5, characterised in that 1,1,1,3,3,3-hexafluoropropene is prepared by carrying out the reaction at 435 to 525°C.

7. Process according to Claims 1 to 6, characterised in that 2-chloro-1,1,1,3,3,3-hexafluoropropane is prepared by carrying out the process at 325 to 415°C.

8. Process according to Claims 1 to 7, characterised in that CF$_3$-CCl$_2$-CF$_3$ and/or CF$_3$-CHCl-CF$_3$ separated off from the reaction mixture is hydrogenated catalytically to give 1,1,1,3,3,3-hexafluoropropane.

9. Process according to Claim 8, characterised in that transition metals on support materials are used as hydrogenation catalysts.

**Revendications**

1. Procédé de production d'hexafluoropropanes de formule (I)
$$CF_3\text{-}CHX\text{-}CF_3 \qquad (I),$$
dans laquelle
X représente l'hydrogène ou le chlore,
caractérisé en ce qu'on fait réagir de l'hexachloropropène avec du fluorure d'hydrogène en phase gazeuse en présence d'un catalyseur.

2. Procédé suivant la revendication 1, caractérisé en ce qu'on conduit la réaction à une température de 250 à 600°C.

3. Procédé suivant les revendications 1 et 2, caractérisé en ce qu'on utilise par mole d'hexachloropropène 5 à 100 moles de fluorure d'hydrogène.

4. Procédé suivant les revendications 1 à 3, caractérisé en ce qu'on utilise comme catalyseurs des halogénures et/ou des oxydes de métaux et/ou de métaux de transition.

5. Procédé suivant les revendications 1 à 4, caractérisé en ce qu'on utilise des catalyseurs contenant des sels de chrome(III).

6. Procédé suivant les revendications 1 à 5, caractérisé en ce qu'on conduit la réaction à 435-525°C pour la production du 1,1,1,3,3,3-hexafluoropropène.

7. Procédé suivant les revendications 1 à 6, caractérisé en ce qu'il est mis en oeuvre à une température de 325 à 415°C pour la production du 2-chloro-1,1,1,3,3,3-hexafluoropropane.

8. Procédé suivant les revendications 1 à 7, caractérisé en ce qu'on hydrogène en 1,1,1,3,3,3-hexafluoropropane par voie catalytique du $CF_3$-$CCl_2$-$CF_3$ et/ou $CF_3$-$CHCl$-$CF_3$ séparés du mélange réactionnel.

9. Procédé suivant la revendication 8, caractérisé en ce qu'on utilise comme catalyseurs d'hydrogénation des métaux de transition fixés sur des supports.